# EUROPEAN PATENT SPECIFICATION

(11) **EP 0 837 039 B1**
(45) Date of publication and mention of the grant of the patent: **07.07.1999**
(21) Application number: 96942591.7
(22) Date of filing: 20.12.1996
(51) Int. Cl.: C02F 3/10, C12N 11/00

(54) **CONSTRUCTION OF MATERIAL FOR CARRIER STRUCTURE FOR GROUP OF EFFECTIVE MICROORGANISMS AND GROUP OF PRODUCTS**
MATERIALKONSTRUKTION FÜR DIE TRÄGERSTRUKTUR EINER GRUPPE VON EFFEKTIVEN MIKROORGANISMEN UND PRODUKTEN
REALISATION D'UN MATERIAU DESTINE A LA STRUCTURE PORTEUSE D'UN GROUPE DE MICROORGANISMES ACTIFS ET D'UN GROUPE DE PRODUITS

(30) Priority: 09.02.1996 JP 4803996
(43) Date of publication of application: 22.04.1998
(73) Proprietor: Tanabe Industry Ltd., Nigata 949-03 (JP)
(72) Inventor: YOSHIOKA, Kouichi, Tanabe Industry Ltd., Nishikubiki-gun, Niigata 949-03 (JP)
(74) Representative: Sanderson, Michael John
(86) International application number: JP9603725
(87) International publication number: WO9729051

(56) References cited:
- JP-A- 4 247 297
- JP-A- 7 132 284
- JP-A- 8 000 181
- JP-A- 8 010 781
- JP-A- 8 109 270
- JP-A- 8 133 814
- JP-A- 8 169 745

## Description

### TECHNICAL FIELD

The present invention relates to a material of effective microbe carrier structure and products made of this material, which maintain an active level of microbe utilized in water treatment such as water purification and waste water treatment, decontaminating treatment or recycling, e.g., composting of garbage, agricultural wastes and livestock wastes.

### TECHNICAL BACKGROUND

Conventionally, carriers for maintaining an active level of microbe, growing microbe and efficiently utilizing microbe are made of ceramics, formed in various shapes, e.g. a porous shape and a hollow shape such as a snail.

### DISCLOSURE OF THE INVENTION

The conventional carriers, particularly, ceramic carriers have been invented, researched and developed, including their shapes, and their qualities have been quite improved.

However, carriers cannot stably carry a large quantity of microbe, which disturbs the sufficient utilization of microbe.

Further, there are various restrictions on usable areas and shapes of carriers.

It is an object of the present invention to provide a material of effective microbe carrier structure, which overcomes the problems and restrictions of the conventional carriers, and which is utilized as a material of a purifying equipment to use the environmental purifying ability of microbe. Further, it is another object of the present invention to provide a material of effective microbe carrier structure and products made of this material, in which tourmaline that is a silicate mineral proven to have the environmental purifying ability and effective microbe constituted by a group of useful fermentative bacteria and a group of useful composite bacteria including kinds of microbe having different characteristics such as anaerobic microbe and aerobic microbe are used to enhance the ability of recycling wastes.

The present invention can be used as an equivalent for ready-mixed concrete by mixing tourmaline particles and powder classified by size which are made by crushing tourmaline ore that is a silicate mineral, an effective microbe fermentative material produced by mixing effective microbe dilute solution constituted by a group of useful fermentative bacteria and a group of useful composite bacteria including kinds of microbe having different characteristics such as anaerobic microbe and aerobic microbe and molasses into an organic substance such as rice bran, fermenting and drying the mixed organic substance, and cement.

### BRIEF DESCRIPTION OF DRAWINGS

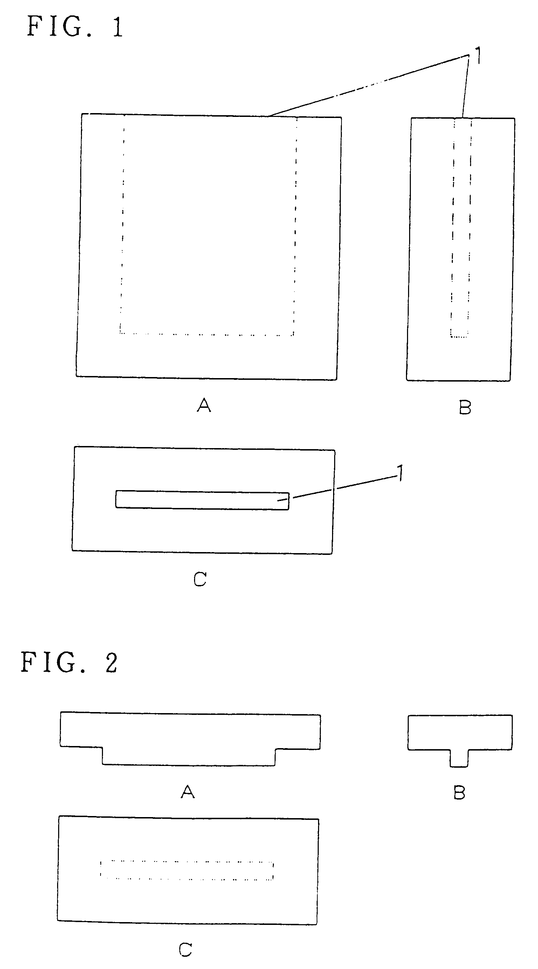

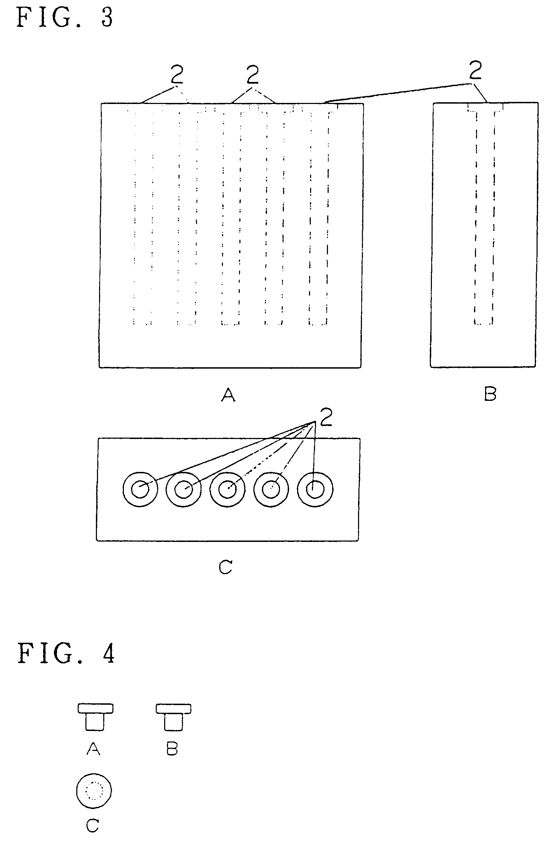

Fig. 1 is a product of effective microbe carrier structure having storage hollow plate structure for regularly supplying effective microbe according to one embodiment; Fig. 2 is a lid of the product of effective microbe carrier structure having storage hollow plate structure for regularly supplying effective microbe according to one embodiment; Fig. 3 is a product of effective microbe carrier structure having storage tube-shaped hollow structure for regularly supplying effective microbe according to one embodiment; Fig. 4 is a detent plug of the product of effective microbe carrier structure having storage tube-shaped hollow structure for regularly supplying effective microbe shown in Fig. 3, according to one embodiment.

### DESCRIPTION OF REFERENCE NUMERALS

Reference numeral 1 is hollow plate structure for storing effective microbe; reference numeral 2 is tube-shaped hollow structure for storing effective microbe.

### BEST MODE OF EMBODYING THE PRESENT INVENTION

A material of effective microbe carrier structure defined in claim 1 can be used as an equivalent for ready mixed concrete by mixing mixture of cement, coarse particles having 1mm-20mm length, made by crushing tourmaline ore, fine particles of tourmaline having 100 µm-1mm length, made by crushing tourmaline ore and sifting with a sieve of 100 µm, of which the weight ratio is 1:2-3:2-3:2-3, an effective microbe (hereinafter called EM) fermentative material, produced by mixing effective microbe dilute solution constituted by a group of useful fermentative bacteria and a group of useful composite bacteria including kinds of microbe having different characteristics such as anaerobic microbe and aerobic microbe, and molasses into an organic substance such as rice bran having ten to hundred percents of the quantity of the mixture, fermenting and drying the mixed organic substance, solution of effective microbe and molasses having equivalent quantity used in the production of the effective microbe fermentative material, and predetermined quantity of water.

The standard method for producing the fermentation material of effective microbe is, for example, mixing 300cc of effective microbe constituted by a group of effective fermentation bacteria and a group of effective composite bacteria including kinds of microbe having different characteristics such as anaerobic microbe and aerobic microbe and 600cc of molasses into 15 litters of water having approximately 40 °C, leaving the mixture 10 to 15 hours, agitating the mixture and 100kg-rice bran in an agitator for 30 to 40 minutes, maturing the mixture in a curing box for 48 to 72 hours and drying the mixture.

"solution of effective microbe and molasses having equivalent quantity used in the production of the effective microbe fermentative material" which is a subject matter of the material of effective microbe carrier structure defined in claim 1 means that the quantity of effective microbe and the molasses as low materials of the effective microbe fermentative material, calculated based on the composition rate of EM and molasses, which is shown in the standard method for producing the effective microbe fermentative material.

It should be noted that the rice bran is used as a base material of EM fermentative material but sawdust, starch pulp, common millet, powder of seeds of sunflower or the like may be used.

Tourmaline ore that is a silica mineral has been known as an ore generating weak current other than jewelry but there was no effective way of use of tourmaline and the most tourmaline not used as jewelry was abandoned. However, recently, abilities of water purification and air purification of the tourmaline have been studied, and the tourmaline has been known as an inexpensive environmental improving material. In particular, the ability of the environmental purification and the ability of growing the microbe, e.g., for purifying the water or breaking down the garbage with the generating weak current have been remarkably observed.

The material of effective microbe carrier structure of the present invention is formed by mixing the sufficient EM fermentative material with the cement to be concrete, so that the EM fermentative material functions to form a porous portion as in a case of formation of fire insulating bricks that saw dusts are kneaded in diatomaceous earth and burned to be formed as a porous portion.

In the present invention, in the case of claims 1, 2 and 3, the porous portion is not formed by burning the EM fermentative material similar to the fire insulating bricks but formed by the chemical reaction of cement and water, so that the remainder of the EM fermentative material is the nutrition supply for growing the ridging of effective microbe having large fecundity under the sever environment, and after the nutrition is consumed, it becomes the porous portion as a carrier for the effective microbe. Thereafter, it incorporates the water and wastes as the nutrition and breaks them up for recycling.

In a case of manufacturing a ceramic carrier, in general, the ceramic material having characteristics suitable for carriers are shaped and burned. In this case, if the EM fermentative material is mixed during shaping, the effective microbe perishes due to the burning temperature of hundreds Celsius degrees.

In the case of claims 1 and 2 of the present invention, the particles and powder mixed in the formed concrete activate the effective microbe included in the fermentative material of effective microbe, support the growth of the microbe and perform the environmental purification.

In claim 2, the tourmaline resin pellet made by mixing tourmaline powder into a synthetic resin is used because the tourmaline resin pellet has the same ability as the tourmaline particles or powder and it is easily handled depending on the conditions for the usage of material of effective microbe carrier structure of the present invention.

In the material of effective microbe carrier structure according to claim 3, sand, gravel or crushed stone is used. This is because it shows the sufficient effects to mix the EM fermentative material to the standard concrete base material, depending on the usage and costs.

In order to avoid the outbreaks of red tide and water-bloom in the lakes and marshes due to the water pollution and excess nutrition, the kinds of ceramic carriers including effective microbe are thrown into the lakes and marshes.

In this case, the effective microbe needs to be regularly supplied every spring but it is hard to collect the carriers from the lakes and marshes due to their topography.

Further, it is difficult to grow and maintain the effective microbe in the swift current for long period of time.

Furthermore, in the case of the lakes and marshes arranged in golf courses or gardens, it is important to keep the beautiful sights in the light of surroundings.

In the above cases, utilizing the product defined in one of claims 4 to 6, claim 1 and claim 2 of the present invention solves the above-described problems.

To utilize a material of effective microbe carrier structure defined in one of claims 1 to 3 as a constituting material of a drainage tank for gardens and golf courses can increase the water quality preservation under the natural design of facility.

For example, the material of effective microbe carrier structure is formed into a shape of fountain according to claim 6 of the present invention, and the water of the lakes and marshes together with service water are used in circulation. If during circulation, circulated water touches with the effective microbe carried in the product of the material of effective microbe carrier structure, the material of effective microbe carrier structure is effective.

It should be noted that the shape of the product of the effective microbe carrier structure according to claim 4 can be formed in any three-dimensional shapes in accordance with its usage and conditions for utilization.

Further, it does not have to be formed into the hollow structure for storing effective microbe.

If an inner lining layer made of a material of effective microbe carrier structure according to one of claims 8 to 10 is provided in waste water treatment facilities including small-scale, medium-scale and large-scale facilities such as a purifying facility and a rural waste water treatment equipment, and flora and fauna waste treatment facilities, the abilities of the environment purifying facilities drastically improve.

Figs. 1 to 4 showing the one embodiment are shown with a rectangular block which is the simplest image, for clarifying the image of the product of effective microbe carrier hollow structure according to the present invention.

In the embodiment shown in Fig. 1, the hollow plate portion can be formed and shaped using the molding box, similar to the manufacture of general concrete blocks. The hollow portion shown in Fig. 1 is normally sealed with a lid having protrusion shown in Fig. 2 for tightly covering the upper opening.

In order to supply the effective microbe or the EM fermentative material, the lid shown in Fig. 2 is removed. Alternately, an inlet suitable for the detent plug shown in Fig. 4 is provided to the lid shown in Fig. 2 and the lid is fixed to the hollow portion.

In the case of the embodiment shown in Fig. 3, the hollow plate portion can also be formed and shaped using the molding box, similar to the manufacture of general concrete blocks. The hollow portion shown in Fig. 3 is normally sealed with a detent plug shown in Fig. 4 for tightly covering the upper opening.

In order to supply the effective microbe or the fermentation material of effective microbe, similar to the product of effective microbe carrier structure having hollow plate structure shown in Fig. 1, the detent plug shown in Fig. 4 is removed.

Installed location for the product of effective microbe carrier hollow structure defined in one of claims 5 and 6 should carefully be considered. In particular, the product for carrying and growing the effective microbe is made of the material of effective microbe carrier structure in accordance with claims 1 to 3, and the portion of the product in contact with the drainage and polluted water should be porous and the remaining portion can be formed in any shapes.

The composition rate of the constituting material defined in one of claims 1 to 3 of the present invention is ranged flexibly. This is because basically the strength is not required like the buildings when the effective microbe carrier structure is manufactured in accordance with the present invention. In the effective microbe structure according to the present invention, only the strength of the effective microbe structure itself needs to be considered since it is utilized in the facilities that satisfy the requisite strength to increase the ability of recycling. Accordingly, the composition rate of the constituting material is regulated on the basis of the range between the common proportion and fine proportion of the concrete mixing, considering the deterioration of the strength due to the porous portion.

### INDUSTRIAL APPLICABILITY

Owing to the utilization of products of a material of effective microbe carrier structure according to the present invention, the water pollution of rivers, lakes and marshes is reduced, and the abilities of waste water treatment facilities including small-scale, medium-scale and large-scale facilities such as a purifying facility and a rural waste water treatment equipment are improved, and the abilities of recycling apparatus for recycling garbage, flora and fauna wastes are improved.

## Claims

1. A material of effective microbe carrier structure produced by mixing,
mixture of cement, coarse particles having 1mm-20mm length, made by crushing tourmaline ore, fine particles of tourmaline having 100 µm-1mm length, made by crushing tourmaline ore, and powder of tourmaline, made by crushing tourmaline ore and sifting with a sieve of 100 µm, weight ratio of said concrete, said coarse particles, said fine particles and said powder being 1:2-3:2-3:2-3;
an effective microbe fermentative material, produced by mixing effective microbe dilute solution constituted by a group of useful fermentative bacteria and a group of useful composite bacteria including kinds of microbe having different characteristics such as anaerobic microbe and aerobic microbe, and molasses into an organic substance such as rice bran having ten to hundred percents of the quantity of said mixture, fermenting and drying the mixed organic substance;
solution of effective microbe and molasses having equivalent quantity used in the production of said effective microbe fermentative material; and
predetermined quantity of water;
said material of effective microbe carrier structure being able to use as ready-mixed concrete.

2. A material of effective microbe carrier structure according to claim 1, wherein said coarse particles of tourmaline having 1mm to 20mm length is substituted by a tourmaline resin pellet, formed by mixing tourmaline powder into a synthetic resin.

3. A material of effective microbe carrier structure produced by mixing,
mixture of cement, coarse particles of sand having 1mm-20mm length, fine particles of sand or crushed stone having 100 µm-1mm length, and powder of sand or crushed stone made by crushing and sifting with a sieve of 100µm, weight ratio of said concrete, said coarse particles, said fine particles and said powder being 1:2-3:2-3:2-3;
an effective microbe fermentative material, produced by mixing effective microbe dilute solution constituted by a group of useful fermentative bacteria and a group of useful composite bacteria including kinds of microbe having different characteristics such as anaerobic microbe and aerobic microbe, and molasses into an organic substance such as rice bran having ten to hundred percents of the quantity of said mixture, fermenting and drying the mixed organic substance;
solution of effective microbe and molasses having equivalent quantity used in the production of said effective microbe fermentative material; and
predetermined quantity of water;
said material of effective microbe carrier structure being able to be used as ready-mixed concrete.

4. A product of effective microbe carrier structure comprising:
storage hollow structure, made of a material of effective microbe carrier structure according to any one of claims 1 to 3, for regularly supplying effective microbe constituted by a group of useful fermentative bacteria and a group of useful composite bacteria including kinds of microbe having different characteristics such as anaerobic microbe and aerobic microbe.

5. A product of effective microbe carrier structure comprising:
storage hollow structure, made of a material of effective microbe carrier structure according to any one of claims 1 to 3, for regularly supplying effective microbe constituted by a group of useful fermentative bacteria and a group of useful composite bacteria including kinds of microbe having different characteristics such as anaerobic microbe and aerobic microbe; and
exterior having ornaments of natural rocks.

6. A product of effective microbe carrier structure comprising:
storage hollow structure, made of a material of effective microbe carrier structure according to any one of claims 1 to 3, for regularly supplying effective microbe constituted by a group of useful fermentative bacteria and a group of useful composite bacteria including kinds of microbe having different characteristics such as anaerobic microbe and aerobic microbe; and
exterior having a shape of a garden ornament such as a garden lantern and fountain.

7. An internal lining layer for a waste water treatment facility including small-scale, medium-scale and large-scale facilities such as a purifying facility and a rural waste water treatment equipment comprising;
storage hollow structure, made of a material of effective microbe carrier structure according to any one of claims 1 to 3, for regularly supplying effective microbe constituted by a group of useful fermentative bacteria and a group of useful composite bacteria including kinds of microbe having different characteristics such as anaerobic microbe and aerobic microbe.

8. An internal lining layer for a flora and fauna waste treatment facility including facilities for treating garbage, agricultural wastes and livestock wastes comprising;
storage hollow structure, made of a material of effective microbe carrier structure according to any one of claims 1 to 3, for regularly supplying effective microbe constituted by a group of useful fermentative bacteria and a group of useful composite bacteria including kinds of microbe having different characteristics such as anaerobic microbe and aerobic microbe.

9. An internal lining layer for a waste water treatment facility including small-scale, medium-scale and large-scale facilities such as a purifying facility and a rural waste water treatment equipment comprising a material of effective microbe carrier structure according to any one of claims 1 to 3.

10. An internal lining layer for a flora and fauna waste treatment facility including facilities for treating garbage, agricultural wastes and livestock wastes comprising a material of effective microbe carrier structure according to any one of claims 1 to 3.

## Patentansprüche

1. Material mit wirksamer Mikrobenträgerstruktur, erhalten durch Zusammenmischen
eines Gemisches von Zement, durch Zerkleinern von Turmalinerz hergestellten Grobteilchen mit einer Länge von 1 bis 20 mm, durch Zerkleinern von Turmalinerz hergestellten Feinteilchen mit einer Länge von 100 µm bis 1 mm und durch Zerkleinern von Turmalinerz und Sieben durch ein 100 µm-Sieb hergestelltem Turmalinpulver, wobei das Gewichtsverhältnis zwischen Zement, Grobteilchen, Feinteilchen und Pulver 1:2-3:2-3:2-3 ist;
eines wirksamen Mikrobenfermentiermaterials, erzeugt durch Einmischen einer verdünnten wirksamen Mikrobenlösung, die aus einer Gruppe nützlicher fermentativer Bakterien und einer Gruppe nützlicher Komposit-Bakterien besteht, die Mikrobenarten mit unterschiedlichen Eigenschaften umfassen, wie anaerobe Mikroben und aerobe Mikroben, sowie Melasse in eine organische Substanz wie Reiskleie mit 10 bis 100 % der Menge des Gemisches, Fermentieren und Trocknen der gemischten organischen Substanz, wobei die
wirksame Mikrobenlösung und die Melasse in äquivalenten Mengen für die Herstellung des wirksamen Mikrobenfermentationsmaterials verwendet werden; und
einer vorbestimmten Wassermenge;
wobei das Material mit wirksamer Mikrobenträgerstruktur als Beton-Fertiggemisch verwendet werden kann.

2. Material mit wirksamer Mikrobenträgerstruktur nach Anspruch 2, worin die Turmalin-Grobteilchen mit 1 bis 20 mm Länge durch ein durch Einmischen von Turmalinpulver in ein Kunstharz hergestelltes Turmalin-Harzpellet ersetzt sind.

3. Material mit wirksamer Mikrobenträgerstruktur, erhalten durch Zusammenmischen
eines Gemisches von Zement, Sand-Grobteilchen mit einer Länge von 1 bis 20 mm, Feinteilchen aus Sand oder zerkleinertem Stein mit einer Länge von 100 µm bis 1 mm und durch Zerkleinern und Sieben durch ein 100 µm-Sieb hergestelltem Pulver aus Sand oder zerkleinertem Stein, wobei das Gewichtsverhältnis zwischen dem Beton, den groben Teilchen, den feinen Teilchen und dem Pulver 1:2-3:2-3:2-3 ist;
eines wirksamen Mikrobenfermentiermaterials, erzeugt durch Einmischen einer verdünnten wirksamen Mikrobenlösung, die aus einer Gruppe nützlicher fermentativer Bakterien und einer Gruppe nützlicher Komposit-Bakterien besteht, die Mikrobenarten mit unterschiedlichen Eigenschaften umfassen, wie anaerobe Mikroben und aerobe Mikroben, sowie Melasse in eine organische Substanz wie Reiskleie mit 10 bis 100 % der Menge des Gemisches, Fermentieren und Trocknen der gemischten organischen Substanz, wobei die
wirksame Mikrobenlösung und die Melasse in äquivalenten Mengen für die Herstellung des wirksamen Mikrobenfermentationsmaterials verwendet werden; und
einer vorbestimmten Wassermenge;
wobei das Material der wirksamen Mikrobenträgerstruktur als Beton-Fertiggemisch verwendet werden kann.

4. Erzeugnis mit wirksamer Mikrobenträgerstruktur, umfassend:
eine hohle Lagerstruktur, bestehend aus einem Material mit wirksamer Mikrobenträgerstruktur nach einem der Ansprüche 1 bis 3, zum regelmäßigen Zuführen wirksamer Mikroben, die aus einer Gruppe nützlicher fermentativer Bakterien und einer Gruppe nützlicher Komposit-Bakterien bestehen, die Mikrobenarten mit unterschiedlichen Eigenschaften, wie anaerobe Mikroben und aerobe Mikroben, umfassen.

5. Erzeugnis mit wirksamer Mikrobenträgerstruktur, umfassend:
eine hohle Lagerstruktur aus einem Material mit wirksamer Mikrobenträgerstruktur nach einem der Ansprüche 1 bis 3, zum regelmäßigen Zuführen wirksamer Mikroben, die aus einer Gruppe nützlicher fermentativer Bakterien und einer Gruppe nützlicher Komposit-Bakterien bestehen, die Mikrobenarten mit unterschiedlichen Eigenschaften, wie anaerobe Mikroben und aerobe Mikroben, umfassen; und
ein Äußeres mit Verzierungen aus Natursteinen.

6. Erzeugnis mit wirksamer Mikrobenträgerstruktur, umfassend:
eine hohle Lagerstruktur aus einem Material mit wirksamer Mikrobenträgerstruktur nach einem der Ansprüche 1 bis 3, zum regelmäßigen Zuführen wirksamer Mikroben, die aus einer Gruppe nützlicher fermentativer Bakterien und einer Gruppe nützlicher Komposit-Bakterien bestehen, die Mikrobenarten mit unterschiedlichen Eigenschaften, wie anaerobe Mikroben und aerobe Mikroben, umfassen; und
ein Äußeres in Gestalt eines Gartenziergegenstands, wie einer Gartenlaterne oder eines Brunnens.

7. Innenauskleidungsschicht für eine Abwasserbehandlungsanlage, einschließlich Anlagen kleiner, mittlerer und großer Größe, wie eine Reinigungsanlage und eine Abwasserbehandlungsausrüstung für die Landwirtschaft, umfassend:
eine hohle Lagerstruktur aus einem Material mit wirksamer Mikrobenträgerstruktur nach einem der Ansprüche 1 bis 3 zum regelmäßigen Zuführen wirksamer Mikroben, die aus
einer Gruppe nützlicher fermentativer Bakterien und einer Gruppe nützlicher Komposit-Bakterien bestehen, die Mikrobenarten mit unterschiedlichen Eigenschaften umfassen, wie anaerobe Mikroben und aerobe Mikroben.

8. Innenauskleidungsschicht für eine Abfallbehandlungsanlage für Flora und Fauna, einschließlich Anlagen zur Behandlung von Müll, landwirtschaftlichen Abfällen und Tiermist, umfassend:
eine hohle Lagerstruktur aus einem Material mit wirksamer Mikrobenträgerstruktur nach einem der Ansprüche 1 bis 3 zum regelmäßigen Zuführen wirksamer Mikroben, die aus einer Gruppe nützlicher fermentativer Bakterien und einer Gruppe nützlicher Komposit-Bakterien bestehen, die Mikrobenarten mit unterschiedlichen Eigenschaften umfassen, wie anaerobe Mikroben und aerobe Mikroben.

9. Innenauskleidungsschicht für eine Abwasserbehandlungsanlage, einschließlich Anlagen kleiner, mittlerer und großer Größe, wie einer Reinigungsanlage und einer Abwasserbehandlungsausrüstung für die Landwirtschaft, die ein Material mit wirksamer Mikrobenträgerstruktur nach einem der Ansprüche 1 bis 3 umfaßt.

10. Innenauskleidungsschicht für eine Abfallbehandlungsanlage für Flora und Fauna, einschließlich Anlagen zur Behandlung von Müll, landwirtschaftlichen Abfällen und Tiermist, die ein Material mit wirksamer Mikrobenträgerstruktur nach einem der Ansprüche 1 bis 3 umfaßt.

## Revendications

1. Matériau d'une structure porteuse de microbes actifs produit en mélangeant,
un mélange de ciment, de particules grossières ayant 1mm-20mm de long, obtenues en broyant un minerai de tourmaline, de particules fines de tourmaline ayant une longueur de 100 µm-1mm, obtenues en broyant un minerai de tourmaline, et de poudre de tourmaline, obtenue en broyant du minerai de tourmaline et en tamisant avec un tamis de 100 µm, le rapport pondéral dudit béton, desdites particules grossières, desdites particules fines et de ladite poudre étant de 1:2-3:2-3:2-3 ;
un matériau actif de fermentation de microbes actifs, produit par mélange d'une solution diluée de microbes actifs constituée d'un groupe de bactéries utiles de fermentation et d'un groupe de bactéries composites utiles comprenant des types de microbes ayant des caractéristiques différentes, comme un microbe anaérobie et un microbe aérobie, et des mélasses dans une substance organique comme du son de riz ayant dix à cent pour cent de la quantité dudit mélange, fermentation et séchage de la substance organique mélangée ;
une solution du microbe actif et des mélasses ayant une quantité équivalente, utilisée dans la production dudit matériau de fermentation de microbes actifs ; et
une quantité prédéterminée d'eau ;
ledit matériau de structure porteuse de microbes actifs pouvant être utilisé en tant que béton prêt mélangé.

2. Matériau d'une structure porteuse de microbes actifs selon la revendication 1, où lesdites particules grossières de tourmaline, ayant 1mm à 20mm de long est substitué par une boulette de résine de tourmaline, formée en mélangeant de la poudre de tourmaline dans une résine synthétique.

3. Matériau d'une structure porteuse de microbes actifs produite en mélangeant
un mélange de ciment, de particules grossières de sable ayant 1mm-20mm de long, de particules fines de sable ou de pierre broyée ayant 100 µm-1mm de long, et de poudre de sable ou de pierre broyée obtenue en broyant et en tamisant avec un tamis de 100 µm, le rapport pondéral dudit béton, desdites particules grossières, desdites particules fines de ladite poudre étant de 1:2-3:2-3:2-3 ;
un matériau de fermentation de microbes actifs, produit par mélange une solution diluée de microbes actifs constituée d'un groupe de bactéries utiles en fermentation et un groupe de bactéries composites utiles, comprenant des types de microbe ayant des caractéristiques différentes comme un microbe anaérobie et un microbe aérobie, et des mélasses dans une substance organique comme du son de riz ayant dix à cent pour cent de la quantité dudit mélange, fermentation et séchage de la substance organique mélangée ;
une solution du microbe actif et des mélasses ayant une quantité équivalente utilisée dans la production dudit matériau de fermentation de microbes actifs ; et
une quantité prédéterminée d'eau ;
ledit matériau de structure porteuse de microbes actifs pouvant être utilisé en tant que béton prêt mélangé.

4. Produit d'une structure porteuse de microbes actifs, comprenant :
une structure creuse de stockage, faite d'un matériau d'une structure porteuse de microbes actifs selon l'une quelconque des revendications 1 à 3, pour fournir régulièrement un microbe actif constitué d'un groupe de bactéries utiles en fermentation et un groupe de bactéries composites utiles comprenant des types de microbe ayant des caractéristiques différentes comme un microbe anaérobie et un microbe aérobie.

5. Produit d'une structure porteuse de microbes actifs comprenant :
une structure creuse de stockage, faite d'un matériau d'une structure porteuse de microbes actifs selon l'une quelconque des revendications 1 à 3, pour fournir régulièrement un microbe actif constitué d'un groupe de bactéries utiles en fermentation et d'un groupe de bactéries composites utiles comprenant des types de microbes ayant des caractéristiques différentes comme un microbe anaérobie et un microbe aérobie ; et
un extérieur ayant des décorations de roches naturelles.

6. Produit de structure porteuse de microbes actifs comprenant :
une structure creuse de stockage, faite d'un matériau d'une structure porteuse de microbes actifs selon l'une quelconque des revendications 1 à 3, pour fournir régulièrement un microbe actif constitué d'un groupe de bactéries utiles en fermentation et d'un groupe de bactéries composites utiles comprenant les types de microbe ayant différentes caractéristiques comme un microbe anaérobie et un microbe aérobie ; et
un extérieur ayant la forme d'une décoration de jardin comme une lanterne de jardin et une fontaine.

7. Couche de revêtement interne pour une installation de traitement des eaux usées comprenant des installations à petite échelle, à moyenne échelle et à grande échelle comme une installation de purification et un équipement de traitement des eaux usées rurales comprenant ;
une structure creuse de stockage, faite d'un matériau d'une structure porteuse de microbes actifs selon l'une quelconque des revendications 1 à 3, pour fournir régulièrement un microbe actif constitué d'un groupe de bactéries utiles en fermentation et d'un groupe de bactéries composites utiles comprenant des types de microbe ayant différentes caractéristiques comme un microbe anaérobie et un microbe aérobie.

8. Une couche de revêtement interne pour une installation de traitement des déchets de flore et de faune, comprenant des installations pour le traitement des ordures, des déchets de l'agriculture et des déchets vivants comprenant ;
une structure creuse de stockage, faite d'un matériau d'une structure porteuse de microbes actifs selon l'une quelconque des revendications 1 à 3, pour fournir régulièrement un microbe actif constitué d'un groupe de bactéries utiles en fermentation et d'un groupe de bactéries composites utiles comprenant des types de microbe ayant différentes caractéristiques comme un microbe anaérobie et un microbe aérobie.

9. Une couche de revêtement interne pour une installation de traitement des eaux usées comprenant des installations à petite échelle, à moyenne échelle et à grande échelle et une installation de purification et un équipement de traitement des eaux usées rurales, comprenant un matériau d'une structure porteuse de microbes actifs selon l'une quelconque des revendications 1 à 3.

10. Couche de revêtement interne pour une installation de traitement des déchets de flore et de faune, comprenant des installations pour le traitement des ordures, des déchets de l'agriculture et des déchets vivants comprenant un matériau d'une structure porteuse de microbes actifs selon l'une quelconque des revendications 1 à 3.
